# EUROPÄISCHE PATENTANMELDUNG

(11) **EP 3 381 422 A1**
(43) Veröffentlichungstag der Anmeldung: **03.10.2018**
(21) Anmeldenummer: 18158348.5
(22) Anmeldetag: 23.02.2018
(51) Int. Cl.: A61F 13/06, A61F 5/01, A61F 13/08

(54) **MIT OBERFLÄCHENNAHEN NERVEN WECHSELWIRKENDE FUSS- UND/ODER KNÖCHELBANDAGE**

(30) Priorität: 29.03.2017 DE 202017101834 U
(71) Anmelder: FXF GmbH, 92224 Amberg (DE)
(72) Erfinder: FISCHER, Franz, 92224 Amberg (DE)
(74) Vertreter: Benninger, Johannes

(57) **Zusammenfassung**

Die vorliegende Erfindung betrifft einen Bereich eines menschlichen Fußes (10) und/oder Knöchels (12) umfangende und beim Tragen unter elastischer Vorspannung stehende Bandage (14), die an einer zur Hautoberfläche (18) weisenden Innenseite mit wenigstens einer Profilierung (20), noppen- und/oder linienartigen und/oder zumindest abschnittsweise sich krümmenden und/oder mäandrierenden Erhöhung (22) ausgestattet ist und die mit oberflächennahen Nervenarealen in den Bereichen des Fußes (10) und/oder Knöchels (12), die von der Bandage (14) bedeckt sind, eine Wechselwirkung herstellt und/oder auf diese Nervenareale durch mechanische Druckeffekte einwirkt.

## Beschreibung

Die vorliegende Erfindung betrifft eine am menschlichen Fuß bzw. am Knöchel zu tragende Bandage mit den Merkmalen des unabhängigen Anspruchs, die mit oberflächennahen Nervenarealen des Fußes und/oder Knöchels eine Wechselwirkung herstellt.

Die meisten Aspekte der menschlichen Motorik wie etwa das Gehen und Stehen beim Menschen sind erlernt und weitestgehend automatisiert. Dabei sind in der Motorik für die verschiedenen Bewegungsmuster jeweils Programme hinterlegt, die sich nach kürzerer oder längerer Lernphase bewährt haben und nur noch minimal verändert werden. Hierbei überwacht der sog. sensomotorische Regelkreis neben dem optischen System permanent, ob sich der Körper in dem hinterlegten Muster befindet bzw. ob er sich innerhalb der Grenzen des hinterlegten Musters bewegt. Auftretende Abweichungen werden normalerweise sofort korrigiert.

Das propriozeptive System und das taktile System stehen im sensomotorischen Regelkreis an erster Stelle. Das heißt, die Signale der Nerven im Fuß - und hierbei im Speziellen die Nerven der Fußsohle - werden permanent abgefragt, ob die Empfindungen in den belasteten Zonen mit dem gespeicherten Muster oder mit den gespeicherten Mustern übereinstimmen.

Reduziert oder verschlechtert sich bei einem Menschen, bedingt u.a. durch eine Polyneuropathie, die Eigenwahrnehmung, sind von diesen Erscheinungen zuvorderst und im Zeitverlauf zuerst die dünnsten Stellen und die am weitesten entfernten Stellen betroffen, wobei die abnehmende Eigenwahrnehmung hand- und strumpfförmig fortschreitet. Auch verschlechtert sich ca. ab dem siebzigsten Lebensjahr pro Dekade die Situation um einen Anteil von zwischen etwa zwölf und sechzehn Prozent. Dabei konnte festgestellt werden, dass eine spontane Gehgeschwindigkeit unterhalb eines Wertes von ca. 100 cm/s tendenziell auf motorisch-posturale Instabilitäten hinweisen kann. In der Praxis kann eine solche Reduktion der Gehgeschwindigkeit zu Problemen führen, insbesondere bei der Bewältigung alltäglicher Prozesse und Aufgaben. So ist bspw. für das sichere Überqueren eines Fußgängerüberweges zur Querung einer Straße eine Gehgeschwindigkeit von mindestens 120 cm/s nötig. Bei älteren Menschen funktioniert das Gehen auch nicht mehr im selben Maße automatisch, wie dies in jüngeren Lebensjahren typischerweise der Fall ist, sondern es ist eine höhere Aufmerksamkeit für ein stabiles Gehen nötig.

Aus dem Stand der Technik sind unterschiedliche Ansätze bekannt, um funktionelle Dysbalancen des menschlichen Bewegungsapparates auszugleichen. So offenbart die WO 2014/ 041 075 A1 ein Beinbekleidungsstück in Gestalt eines Strumpfes, der das Verletzungsrisiko eines Trägers bei sportlichen Aktivitäten reduzieren helfen und funktionelle Dysbalancen des Bewegungsapparates ausgleichen soll. Um dies zu erreichen, wird ein Kompressionsbereich vorgeschlagen, damit das Beinbekleidungsstück bzw. der Strumpf im getragenen Zustand eine Kompressionswirkung auf das Bein des Trägers ausüben kann. Zudem soll eine reizinduzierende Struktur vorgesehen sein, die im getragenen Zustand an einer dem Bein des Trägers des Beinbekleidungsstücks zugewandten Innenseite des Beinbekleidungsstückes angeordnet ist.

Zudem offenbart die WO 2015/ 135 852 A1 ein weiteres Beinbekleidungsstück mit eingearbeiteten Funktionselementen, welche voneinander verschiedene Höhen und/oder voneinander verschiedene Shore-A-Härten aufweisen sollen. Auch bei diesem Beinbekleidungsstück soll durch die Funktionselemente im getragenen Zustand eine Kompressionswirkung auf den Körper des Trägers des Beinbekleidungsstückes ausgeübt werden.

Diese beiden bekannten Beinbekleidungsstücke mit ihren Kompressionswirkungen basieren im Wesentlichen auf einer Einwirkung auf das propriozeptive System, was sich jedoch durch neuere Untersuchungen für spezielle Problemstellungen als nicht ausreichend effektiv herausgestellt hat. So hat sich gezeigt, dass die vorgeschlagenen Maßnahmen für jüngere Menschen und für Nutzungen bei sportlicher Betätigung durchaus ihre Berechtigung haben, jedoch bspw. bei altersbedingten polyneuropathischen Erscheinungen wenig Wirkung zeigen.

Ein vorrangiges Ziel der vorliegenden Erfindung ist ein verbessertes System zur Linderung polyneuropathischer Erscheinungen und/oder von nachlassender Eigenwahrnehmung zur Verfügung zu stellen, das insbesondere Verbesserungen bei der menschlichen Motorik beim Gehen und Laufen ermöglichen soll.

Dieses Ziel der der Erfindung wird mit dem Gegenstand des unabhängigen Anspruchs erreicht. Merkmale vorteilhafter Weiterbildungen ergeben sich aus den abhängigen Ansprüchen.

Mit dem Gegenstand der Erfindung können im Wesentlichen alters- und krankheitsbedingte Verschlechterungen der Eigenwahrnehmung und die damit auftretende Sturzgefahr und Verschlechterung des Bewegungsablaufes ausgeglichen werden, indem durch Erzeugung eines mechanischen Drucks im Bereich der unteren Extremitäten eine Verbesserung der sensorischen (afferenten) Rückmeldung geschaffen wird. Das heißt vereinfacht, die Einschränkungen beginnen am dünnsten und am weit entferntesten Bereich des Nervs. Wird der Impuls in einem näherliegenden und dickeren Bereich verstärkt, führt das zu einer besseren Raumorientierung und somit zu einem besseren, sicheren und automatisierten Bewegungsablauf. Somit sinkt die Sturzgefahr des betroffenen Menschen. Letztlich soll mit der erfindungsgemäßen Bandage erreicht werden, dass die Pathologie ein Stockwerk höher durch eine Reizverstärkung ausgeglichen wird. Hierbei wird auf das taktile System eingewirkt, weniger auf das propriozeptive System.

Zur Erreichung dieser genannten Ziele schlägt die vorliegende Erfindung eine Bandage vor, die im getragenen Zustand einen Bereich eines menschlichen Fußes und/oder Knöchels umfängt und die beim Tragen unter elastischer Vorspannung steht. Diese Bandage ist an einer zur Hautoberfläche weisenden Innenseite mit wenigstens einer Profilierung, noppen- und/oder linienartigen und/oder zumindest abschnittsweise sich krümmenden und/oder mäandrierenden Erhöhung ausgestattet, die mit oberflächennahen Nervenarealen in den Bereichen des Fußes und/oder Knöchels, die von der Bandage bedeckt sind, eine Wechselwirkung herstellt und/oder auf diese Nervenareale durch mechanische Druckeffekte einwirkt. Damit kann das gewünschte Ziel einer Beeinflussung des menschlichen taktilen Regelkreises im Sinne einer verstärkenden Wirkung auf von der Fußsohle entfernte Nervenabschnitte, die mit der Fußsohle zwar noch verbunden sind, dort aber nicht mehr die gewünschte Sensibilität liefern, erreicht werden.

Die erfindungsgemäße Bandage kann eine Breite von wenigstens drei Zentimeter aufweisen und dabei zumindest teilweise den Knöchelbereich des Fußes überdecken. Wahlweise kann die Bandage auch deutlich breiter sein, bspw. eine Breite von fünf Zentimeter oder mehr aufweisen, ggf. auch über den Umfang mit leicht variierender Breite.

Weiterhin kann die Bandage wahlweise mehrere noppenartige Profilierungen an ihrer zur Hautoberfläche weisenden Innenseite aufweisen. Diese noppenartigen Profilierungen können sich über ein Areal erstrecken, das bei getragener Bandage den engeren Knöchelbereich ausspart und zumindest teilweise um diesen herum verläuft. Zudem kann vorgesehen sein, dass die wenigstens eine Profilierung und/oder Erhöhung durch ein mit der Bandage verbundenes oder dort eingearbeitetes elastisches Element gebildet ist.

Eine weitere Variante der Bandage sieht vor, dass mehrere Profilierungen und/oder Erhöhungen jeweils unterschiedliche Höhen und/oder Breiten und/oder über ihre jeweilige Längserstreckung variierende Höhen und/oder Breiten aufweisen können. Außerdem kann vorgesehen sein, dass mehrere Profilierungen und/oder Erhöhungen jeweils unterschiedliche Elastizitäten und/oder jeweils unterschiedliche Shore-Härten aufweisen können.

Die übrigen Bereiche der Bandage können durch elastisches textiles Material, wahlweise mit eingearbeiteten oder aufgebrachten Zugbändern oder Zugelementen o. dgl. gebildet sein, um eine gewünschte definierte Zugspannung im Umfangsbereich der getragenen Bandage zu erzielen.

Eine weitere sinnvolle Option der erfindungsgemäßen Bandage kann dadurch gebildet sein, dass die Bandage Teil eines Strumpfes ist, oder dass die Bandage am oberen Bund eines Strumpfes mit diesem verbunden oder in diesen eingearbeitet ist. Auch bei dieser Variante der Bandage kann zusätzlich vorgesehen sein, dass der solchermaßen gebildete Strumpf mit weiteren, an einer zur Hautoberfläche weisenden Innenseite Profilierungen, noppen- und/oder linienartigen und/oder zumindest abschnittsweise sich krümmenden und/oder mäandrierenden Erhöhungen ausgestattet ist. In diesem Zusammenhang sei darauf hingewiesen, dass mit der vorliegenden Anmeldung auch ein Strumpf offenbart sein soll, der einen oder mehrere Aspekte oder Ausstattungen aufweist, die oben im Zusammenhang mit der erfindungsgemäßen Bandage genannt wurden. Wenn daher im vorliegenden Zusammenhang von einen Bandage die Rede ist, so kann damit grundsätzlich immer auch ein Strumpf bzw. ein elastisches Beinkleid in Form eines Sockens oder Strumpfes gemeint sein, so dass hiermit klargestellt ist, dass die beiden Begriffe "Bandage" und "Strumpf" an allen Stellen der vorliegenden Anmeldung gegeneinander austauschbar sind; dies gilt für die Schutzansprüche, für die vorliegende Beschreibung, ebenso wie die nachfolgende Figurenbeschreibung, die bevorzugte oder zumindest sinnvolle Ausführungsbeispiele der Erfindung beschreibt.

Wahlweise können elastische Zugbereiche der Bandage und/oder des Strumpfes zur Beaufschlagung verschiedener Abschnitte der Bandage und/oder des Strumpfes mit Zugkräften und/oder mit teilweise differierenden Zugkräften vorgesehen sein, die jeweils in das Material der Bandage eingearbeitet sind. So umfassen die jeweiligen elastischen Zugbereiche eingestrickte oder eingearbeitete Abschnitte mit höheren elastischen Rückzugskräften gegen auf die Bandage wirkende Dehnungskräfte als andere Bereiche oder sind jeweils durch einen solchen eingestrickten Abschnitt gebildet. Die elastischen Zugbereiche bilden Bereiche in der Bandage aus, bei denen die gewünschten erhöhten Rückstell- oder Rückzugskräfte in der Bandage durch eingestrickte bzw. in das Gewebe- bzw. Gestrickmaterial der Bandage eingearbeitete Bereiche aufgebracht werden. Solche Bereiche können mit modernen Strickmaschinen bzw. Rundstrickmaschinen gezielt hergestellt und an gewünschten Stellen der Bandage eingearbeitet werden, so dass dieser die gewünschten elastischen Eigenschaften eingeprägt werden können.

Vorzugsweise kann vorgesehen sein, dass die erfindungsgemäße Bandage zumindest bereichsweise durch Textil gebildet ist, wobei der mindestens eine elastische Zugbereich und/oder weitere elastische Zugbereiche in das Textil aufgearbeitet sind. Der wenigstens eine elastische Zugbereich und/oder weitere elastische Zugbereiche können durch Einstricken in das Textil der Bandage eingearbeitet sein, was sich bspw. durch dafür vorbereitete Strickmaschinen bzw. Rundstrickmaschinen realisieren lässt.

Der mindestens eine elastische Zugbereich und/oder der wenigstens eine weitere elastische Zugbereich sind Teil des gestrickten Textilmaterials der Bandage, wodurch die Haut geschont wird und Hautirritationen vermieden werden.

Vorzugsweise können der mindestens eine elastische Zugbereich und/oder das mindestens eine Beaufschlagungsmittel in das Textil der Bandage eingearbeitet sein, wobei der wenigstens eine elastische Zugbereich und/oder der wenigstens eine weitere elastische Zugbereich und/oder das elastische Band in das Textil eingestrickt oder dergleichen sein können.

Abschließend wird darauf hingewiesen, dass die einzelnen Ausführungsformen der Bandage auch miteinander in beliebiger Weise kombinierbar sind.

Im Folgenden sollen Ausführungsbeispiele die Erfindung und ihre Vorteile anhand der beigefügten Figur näher erläutern. Die Größenverhältnisse der einzelnen Elemente zueinander in der Figur entsprechen nicht immer den realen Größenverhältnissen, da einige Formen vereinfacht und andere Formen zur besseren Veranschaulichung vergrößert im Verhältnis zu anderen Elementen dargestellt sind.
Fig. 1 zeigt eine schematische Ansicht eines menschlichen Fußes mit im Knöchelbereich aufgebrachter zugelastischer Bandage.
Fig. 2A zeigt verschiedene Sensorareale an einer Innenseite eines menschlichen Fußes, die sich für taktile Wechselwirkungen mit einer erfindungsgemäßen Bandage besonders gut eignen.
Fig. 2B zeigt verschiedene Sensorareale an einer Außenseite eines menschlichen Fußes, die sich für taktile Wechselwirkungen mit einer erfindungsgemäßen Bandage besonders gut eignen.

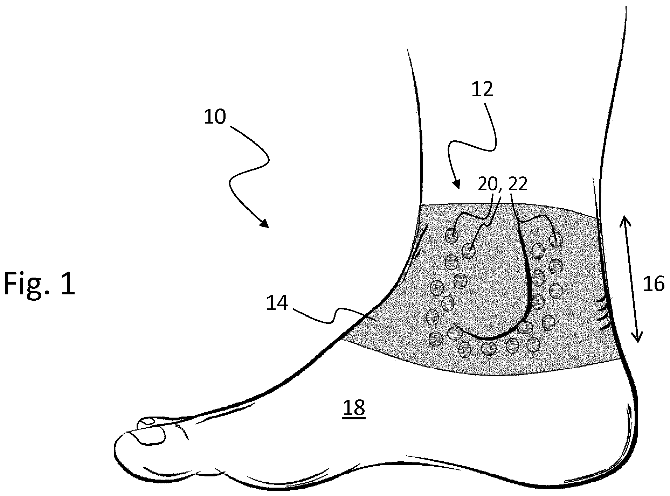

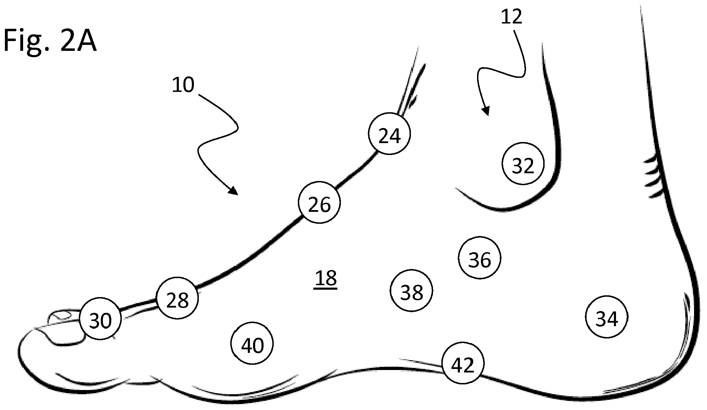

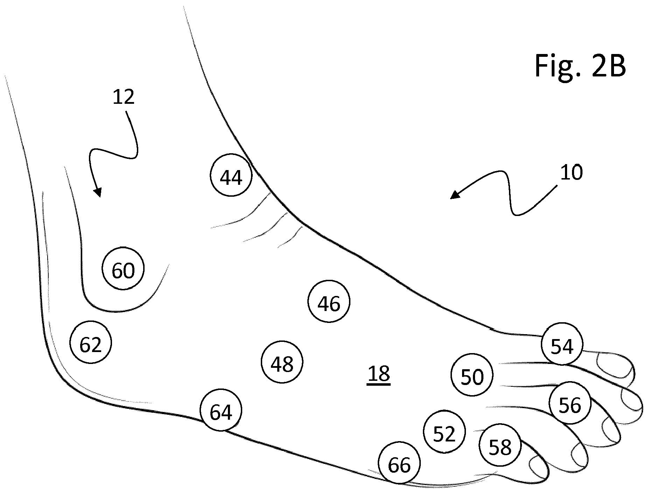

Es sei darauf hingewiesen, dass der Übersicht halber nur diejenigen Bezugsziffern in den Figuren 1, 2A und 2B dargestellt sind, die für die Beschreibung der Figuren erforderlich sind. Die dargestellte Ausführungsform stellt lediglich ein Beispiel dar, wie die erfindungsgemäße Bandage ausgestaltet sein kann und stellt keine abschließende Begrenzung dar.

Die schematische Seitenansicht der Fig. 1 zeigt eine schematische Ansicht einer Innenseite eines menschlichen Fußes 10 mit im Knöchelbereich 12 aufgebrachter zugelastischer Bandage 14. Die Bandage 14 umfängt im getragenen Zustand den Knöchelbereich 12 des Fußes 10 und weist eine Breite 16 von etwa drei bis zwölf Zentimeter oder etwas mehr auf und steht beim Tragen unter elastischer Vorspannung. Die Bandage 14 ist an einer zur Hautoberfläche 18 weisenden Innenseite mit einer Profilierung 20 in Gestalt von zahlreichen noppenartigen Erhöhungen 22 ausgestattet, die mit oberflächennahen Nervenarealen in den Bereichen des Knöchels 12, die von der Bandage 14 bedeckt sind, eine Wechselwirkung herstellen und/oder auf diese Nervenareale durch mechanische Druckeffekte einwirkt. Wahlweise können an der hier nicht gezeigten Fußaußenseite vergleichbare noppenartige Erhöhungen 22 vorgesehen sein, insbesondere im Bereich um den äußeren Knöchel 12.

Mittels der gezeigten Profilierungen 20 kann das gewünschte Ziel einer Beeinflussung des menschlichen taktilen Regelkreises im Sinne einer verstärkenden Wirkung auf von der Fußsohle entfernte Nervenabschnitte, die mit der Fußsohle zwar noch verbunden sind, dort aber nicht mehr die gewünschte Sensibilität liefern, erreicht werden.

Wie es die Fig. 1 erkennen lässt, kann die Bandage 14 insbesondere mehrere noppenartige Erhöhungen 22 an ihrer zur Hautoberfläche 18 weisenden Innenseite aufweisen, die insgesamt die Profilierung 20 an dieser Stelle bilden. Diese noppenartigen Erhöhungen 22 oder Profilierungen 20 können sich über ein Areal erstrecken, das bei getragener Bandage 14 den engeren Knöchelbereich 12 ausspart und zumindest teilweise um diesen herum verläuft, insbesondere U-förmig mit stellenweise doppelter Noppenreihe (vgl. Fig. 1).

Die Profilierung 20 und/oder die Erhöhungen 22 können insbesondere durch mit der Bandage 14 verbundene oder dort eingearbeitete elastische Elemente gebildet sein.

Die übrigen Bereiche der Bandage 14 können durch elastisches textiles Material, wahlweise mit eingearbeiteten oder aufgebrachten Zugbändern oder Zugelementen o. dgl. gebildet sein, um eine gewünschte definierte Zugspannung im Umfangsbereich der getragenen Bandage 14 zu erzielen.

Die Figuren 2A und 2B zeigen verschiedene Sensorareale an einer Innenseite (Fig. 2A) und an einer Außenseite (Fig. 2B) eines menschlichen Fußes 10, die sich für taktile Wechselwirkungen mit einer erfindungsgemäßen Bandage 14 besonders gut eignen. Bei einer solchen Bandage 14 kann es sich wahlweise auch um einen Strumpf (nicht gezeigt) handeln, wie er oben im allgemeinen Teil der Beschreibung als gleichermaßen verwendbare Alternative zur Bandage 14 beschrieben wurde. Auch ein solcher Strumpf kann entsprechende Profilierungen 20 an jeweils sinnvollen Stellen aufweisen.

Hierbei kann es sich bspw. gemäß Fig. 2A an der Fußinnenseite um die nachfolgend genannten Bereiche handeln, wobei jeweils die fachüblichen englischen bzw. lateinischen Bezeichnungen verwendet werden. So bezeichnet die Bezugsziffer 24 den sog. "articulatio talocruralis", die Bezugsziffer 26 die "dorsal base of metatarsus I", die Bezugsziffer 28 die "dorsal metatarsal head I" und die Bezugsziffer 30 die "dorsal distal phalanx III". Weiterhin bezeichnet die Bezugsziffer 32 die "medial malleolus", die Bezugsziffer 34 die "medial calcaneus below malleolus", die Bezugsziffer 36 die "base of os naviculare", die Bezugsziffer 38 die "medial base of metatarsus I", die Bezugsziffer 40 die "medial aspect of metatarsal head I" und die Bezugsziffer 42 den "arch medial".

Außerdem kann es sich bspw. gemäß Fig. 2B an der Fußaußenseite um die nachfolgend genannten Bereiche handeln, wobei wiederum jeweils die fachüblichen englischen bzw. lateinischen Bezeichnungen verwendet werden. So bezeichnet die Bezugsziffer 44 den "articulatio talocruralis", die Bezugsziffer 46 die "dorsal base of metatarsus III", die Bezugsziffer 48 die "dorsal base of metatarsus V", die Bezugsziffer 50 den "dorsal metatarsal head III", die Bezugsziffer 52 den "dorsal metatarsal head V", die Bezugsziffer 54 die "dorsal distal phalanx I", die Bezugsziffer 56 die "dorsal medial phalanx III" und die Bezugsziffer 58 die "dorsal medial phalanx V". Außerdem kann die Bezugsziffer 60 den "lateral malleolus" bezeichnen, die Bezugsziffer 62 den "lateral calcaneus below malleolus", die Bezugsziffer 64 die "lateral base of metatarsus V" und die Bezugsziffer 66 den "lateral aspect of metatarsal head V".

Darüber hinaus können zahlreiche weitere Bereiche einer taktilen Wechselwirkung im Sinne der vorliegenden Erfindung zugänglich sein und hierfür verwendet werden.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezugszeichenliste

- 10: Fuß, menschlicher Fuß
- 12: Knöchelbereich, Knöchel
- 14: Bandage
- 16: Breite (der Bandage)
- 18: Hautoberfläche
- 20: Profilierung
- 22: Erhöhung, noppenartige Erhöhung, elastische Erhöhung
- 24: articulatio talocruralis
- 26: dorsal base of metatarsus I
- 28: dorsal metatarsal head I
- 30: dorsal distal phalanx III
- 32: medial malleolus
- 34: medial calcaneus below malleolus
- 36: base of os naviculare
- 38: medial base of metatarsus I
- 40: medial aspect of metatarsal head I
- 42: arch medial
- 44: articulatio talocruralis
- 46: dorsal base of metatarsus III
- 48: dorsal base of metatarsus V
- 50: dorsal metatarsal head III
- 52: dorsal metatarsal head V
- 54: dorsal distal phalanx I
- 56: dorsal medial phalanx III
- 58: dorsal medial phalanx V
- 60: lateral malleolus
- 62: lateral calcaneus below malleolus
- 64: lateral base of metatarsus V
- 66: lateral aspect of metatarsal head V

## Patentansprüche

1. Einen Bereich eines menschlichen Fußes (10) und/oder Knöchels (12) umfangende und beim Tragen unter elastischer Vorspannung stehende Bandage (14), die an einer zur Hautoberfläche (18) weisenden Innenseite mit wenigstens einer Profilierung (20), noppen- und/oder linienartigen und/oder zumindest abschnittsweise sich krümmenden und/oder mäandrierenden Erhöhung (22) ausgestattet ist, die mit oberflächennahen Nervenarealen in den Bereichen des Fußes (10) und/oder Knöchels (12), die von der Bandage (14) bedeckt sind, eine Wechselwirkung herstellt und/oder auf diese Nervenareale durch mechanische Druckeffekte einwirkt.

2. Bandage nach Anspruch 1, die eine Breite (16) von wenigstens drei Zentimeter aufweist und die zumindest teilweise den Knöchelbereich (12) des Fußes (10) überdeckt.

3. Bandage nach Anspruch 1 oder 2, bei der mehrere noppenartige Profilierungen (20, 22) an ihrer zur Hautoberfläche (18) weisenden Innenseite vorgesehen sind.

4. Bandage nach Anspruch 3, bei der die noppenartigen Profilierungen (20, 22) sich über ein Areal erstrecken, das bei getragener Bandage (14) den engeren Knöchelbereich (12) ausspart und zumindest teilweise um diesen herum verläuft.

5. Bandage nach einem der Ansprüche 1 bis 4, bei der die wenigstens eine Profilierung (20) und/oder Erhöhung (22) durch ein mit der Bandage (14) verbundenes oder dort eingearbeitetes elastisches Element gebildet ist.

6. Bandage nach Anspruch 5, bei der mehrere Profilierungen (20) und/oder Erhöhungen (22) jeweils unterschiedliche Höhen und/oder Breiten und/oder über ihre jeweilige Längserstreckung variierende Höhen und/oder Breiten aufweisen können.

7. Bandage nach Anspruch 5 oder 6, bei der mehrere Profilierungen (20) und/oder Erhöhungen (22) jeweils unterschiedliche Elastizitäten aufweisen können.

8. Bandage nach einem der Ansprüche 1 bis 7, die Teil eines Strumpfes ist, oder die am oberen Bund eines Strumpfes mit diesem verbunden oder in diesen eingearbeitet ist.

9. Bandage nach Anspruch 8, bei welcher der Strumpf mit weiteren, an einer zur Hautoberfläche (18) weisenden Innenseite Profilierungen (20), noppen- und/oder linienartigen und/oder zumindest abschnittsweise sich krümmenden und/oder mäandrierenden Erhöhungen (22) ausgestattet ist.
